# EUROPEAN PATENT APPLICATION

(11) **EP 0 956 838 A2**
(43) Date of publication of application: **17.11.1999**
(21) Application number: 99108979.8
(22) Date of filing: 06.05.1999
(51) Int. Cl.: A61F 5/44

(54) **Body-fluid collecting device**

(30) Priority: 11.05.1998 IT BO980299
(71) Applicant: Medistar S.r.l., 41037 Mirandola (IT)
(72) Inventor: Montanari, Stefano, 41100 Modena (IT)
(74) Representative: Jorio, Paolo

(57) **Abstract**

A collecting device (1) for collecting body fluids withdrawn from a patient by means of a catheter (2) or similar inserted into the patient's body; the device having a collecting bag (3), and a feed tube (4) connecting the collecting bag (3) to the catheter (2); and at least one portion (4b) of the feed tube (4) being made of a bactericidal composite material defined by a matrix of plastic material and a disperse phase of metal salts.

## Description

The present invention relates to a body-fluid collecting device.

More specifically, the present invention relates to a device for collecting urine or any other body fluid withdrawn from a patient's body by means of a catheter or similar; to which application the following description refers purely by way of example.

As is known, currently marketed urine collecting devices comprise a body-fluid collecting bag, and a plastic feed tube having a first end connected to the collecting bag, and a second end with a connector to which the catheter is fitted, so as to feed the urine withdrawn from the patient into the collecting bag.

Collecting devices also comprise a sterilizing member, normally located along the feed tube, to prevent colonies of bacteria from spreading inside the feed tube and the collecting bag.

Urine, in fact, like many other body fluids, depending on the patient's condition, is known to contain in suspension large quantities of bacteria for which the inside of the collecting bag or feed tube is an excellent environment for growth. As serious diseases may be caused by bacteria traveling back up along the feed tube into the patient's body, steps must be taken to prevent colonies of bacteria from spreading inside the collecting device.

In European Patent EP0379507, the sterilizing member of the collecting device comprises a sleeve made of water-soluble glass impregnated with elementary silver or a silver-based compound, and located at the connector to which the catheter is fitted, so that any bacteria in the urine flowing through and over the inside surface of the sleeve are destroyed by the silver present in the glass.

The bactericidal efficiency of a sterilizing member of the above type is directly proportional to the active surface area with which the body fluid is brought into contact, and is therefore only satisfactory for small amounts of fluid, on account of the structural rigidity and fragility typical of glass limiting the axial length of the sleeve.

Other known sterilizing members comprise a cartridge filled with grains of silver or other bactericidal material and located along the feed tube, so that the body fluid flows over the outer surface of the grains of silver and then into the collecting bag.

In this case, the cartridge has an active surface capable of effectively sterilizing large quantities of fluid, but is easily clogged. That is, the urine flowing along the catheter normally contains suspended blood clots which, failing to pass through the cartridge on account of the small-section passages between the grains of silver, collect on the inflow side, thus eventually clogging the cartridge.

It is an object of the present invention to provide a body-fluid collecting device designed to eliminate the aforementioned drawbacks.

According to the present invention, there is provided a collecting device for collecting body fluids withdrawn from a live organism; the collecting device comprising a holding vessel for accumulating said body fluids, and a feed tube having a first end connected to said holding vessel, and a second end connectable to said live organism; said collecting device being characterized in that at least one portion of said feed tube is made of bactericidal composite material.

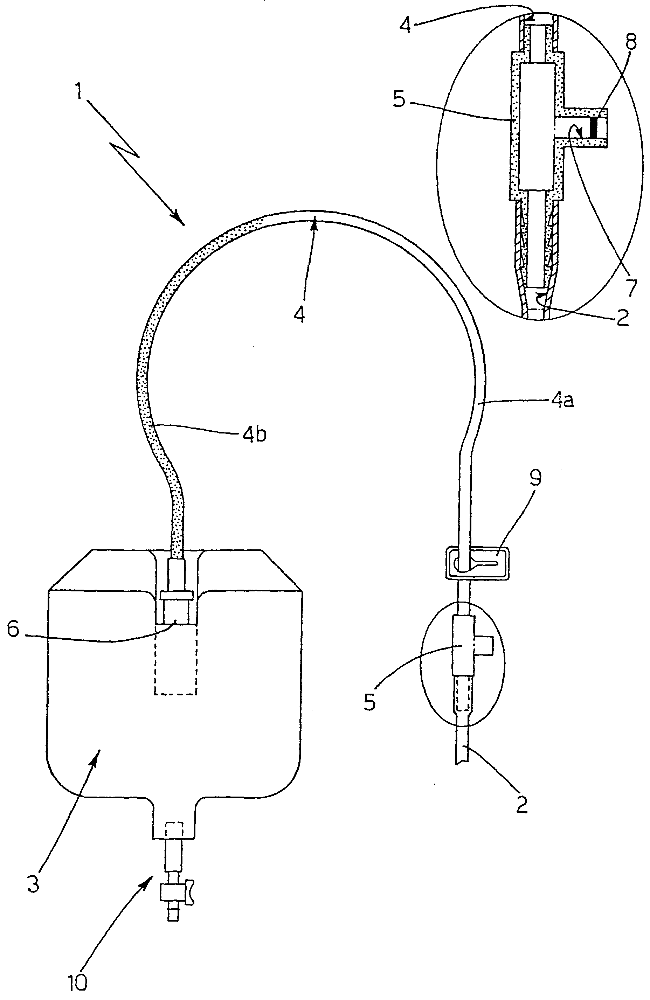

The present invention will be described with reference to the accompanying drawing, which shows a body-fluid collecting device in accordance with the teachings of the present invention.

Number 1 in the accompanying drawing indicates as a whole a body-fluid collecting device for storing body fluids, e.g. urine, withdrawn from a patient, either human or animal, using any withdrawal means, such as a catheter 2 inserted into the patient's body.

Collecting device 1 comprises a body-fluid collecting bag 3; and a feed tube 4 having a first end connected to collecting bag 3, and a second end having a fitting 5 for connection to said withdrawal means, so that the body fluids flow along feed tube 4 into collecting bag 3.

In the example shown, collecting bag 3 has a drip chamber 6, and feed tube 4 communicates directly with drip chamber 6 so that the body fluids flowing inside the tube flow into collecting bag 3 via drip chamber 6.

In the example shown, collecting bag 3 is provided at the bottom with a drain tap 10 by which to empty collecting bag 3.

Collecting device 1 also comprises a withdrawal point by which to withdraw the body fluids flowing along catheter 2 for chemical and physical analysis.

In the example shown, the withdrawal point is formed in fitting 5, which is defined by a tubular body, the lateral wall of which comprises a through hole 7 closed in fluidtight manner by a diaphragm 8 defining the withdrawal point. Diaphragm 8 is perforated easily by a needle or similar to enable troublefree withdrawal of the body fluids from fitting 5.

In the example shown, to assist withdrawal of the body fluids from catheter 2, collecting device 1 comprises a pressure clamp 9 located along feed tube 4, downstream from fitting 5, and which is tightened onto feed tube 4 to cut off the outflow of body fluids and so fill the tube portion between pressure clamp 9 and fitting 5.

With reference to the accompanying drawing, feed tube 4 is divided into two portions made of different materials : the portion directly downstream from fitting 5 - hereinafter referred to as portion 4a - is made of known plastic material; and the portion directly upstream from collecting bag 3 - hereinafter referred to as portion 4b - is made of a bactericidal composite material for preventing colonies of bacteria from spreading inside feed tube 4 and collecting bag 3.

The composite material comprises a matrix of plastic materials, and a disperse phase of bactericidal metal ions. In the example shown, the matrix of plastic materials preferably, but not necessarily, comprises polyethylene, polypropylene, polystyrene or polyvinylchloride, possibly mixed with one another; and the disperse phase of metal ions preferably, but not necessarily, comprises ions of metal salts, such as silver salts, zinc salts, gold salts, platinum salts, lead salts, boron salts, copper salts, calcium salts, silicon salts, etc. The metal salts may, of course, also be mixed with one another in given proportions to obtain greater bactericidal efficiency.

In a first variation not shown, feed tube 4 is made entirely of said bactericidal composite material.

In a further variation not shown, feed tube 4 comprises two or more portions of bactericidal composite material, possibly of different compositions and characteristics.

In the example shown, collecting bag 3 is also made of the same bactericidal composite material as portion 4b of feed tube 4.

Operation of collecting device 1 is easily deducible from the foregoing description with no further explanation required.

The main advantage of collecting device 1 as described and illustrated herein is that of being of extremely straightforward design enabling rapid assembly and a considerable reduction in manufacturing cost.

A further advantage lies in collecting device 1 being free from clogging, and ensuring a degree of sterilization far superior to that of any collecting device currently available on the market. The active surface of collecting device 1 contacted by the body fluids, in fact, is far greater than that of currently marketed collecting devices (especially the variation in which feed tube 4 is made entirely of composite material).

Yet a further advantage of collecting device 1 lies in the high degree of flexibility maintained by feed tube 4, thus enabling the patient to move freely.

Clearly, changes may be made to collecting device 1 as described and illustrated herein without, however, departing from the scope of the present invention.

## Claims

1. A collecting device (1) for collecting body fluids withdrawn from a live organism; the collecting device (1) comprising a holding vessel (3) for accumulating said body fluids, and a feed tube (4) having a first end connected to said holding vessel (3), and a second end connectable to said live organism; said collecting device (1) being characterized in that at least one portion (4b) of said feed tube (4) is made of bactericidal composite material.

2. A device as claimed in Claim 1, characterized in that said bactericidal composite material comprises a matrix of plastic materials and a disperse phase of metal ions.

3. A device as claimed in Claim 2, characterized in that said holding vessel (3) for accumulating body fluids is a body-fluid collecting bag (3).

4. A device as claimed in any one of the foregoing Claims, characterized in that said holding vessel (3) for accumulating said body fluids is made of said bactericidal composite material.

5. A device as claimed in any one of the foregoing Claims, characterized in that said feed tube (4) is made entirely of said bactericidal composite material.

6. A device as claimed in any one of the foregoing Claims, characterized in that said feed tube (4) comprises, at said second end, a fitting (5) connectable to withdrawal means (2) inserted into the body of said live organism.

7. A device as claimed in Claim 6, characterized in that said fitting (5) comprises a withdrawal point (8).

8. A device as claimed in Claim 7, characterized by comprising closing means (9) for closing said feed tube (4) and which are located along the feed tube (4), downstream from said fitting (5).

9. A device as claimed in any one of the foregoing Claims, characterized in that said matrix of plastic materials comprises polypropylene.

10. A device as claimed in any one of Claims 2 to 9, characterized in that said matrix of plastic materials comprises polyvinylchloride.

11. A device as claimed in any one of Claims 2 to 10, characterized in that said matrix of plastic materials comprises polyethylene.

12. A device as claimed in any one of Claims 2 to 11, characterized in that said matrix of plastic materials comprises polystyrene.

13. A device as claimed in any one of the foregoing Claims, characterized in that said disperse phase of metal ions comprises positive metal salt ions.
